# EUROPÄISCHE PATENTSCHRIFT

(11) **EP 3 441 022 B1**
(45) Veröffentlichungstag und Bekanntmachung des Hinweises auf die Patenterteilung: **19.08.2020**
(21) Anmeldenummer: 18000326.1
(22) Anmeldetag: 06.04.2018
(51) Int. Cl.: A61B 17/28, A61B 17/00, A61B 17/12, A61B 17/122, A61B 17/29, A61B 17/16, A61B 17/04

(54) **CHIRURGISCHES INSTRUMENT**
SURGICAL INSTRUMENT
INSTRUMENT CHIRURGICAL

(30) Priorität: 11.08.2017 DE 202017004226 U
(43) Veröffentlichungstag der Anmeldung: 13.02.2019
(73) Patentinhaber: CARDIOMEDICAL GmbH, 30855 Langenhagen (DE)
(72) Erfinder: Wolfgang, Wiedenbein, 30655 Hannover (DE)

(56) Entgegenhaltungen:
- EP-A2- 1 250 891
- DE-T2- 69 632 451
- DE-U1- 20 112 540
- US-A- 5 490 819
- US-A1- 2013 317 313

## Beschreibung

Die vorliegende Erfindung umfasst ein medizinisches Instrument, insbesondere ein chirurgisches Instrument, beispielsweise eine Gefäßklemme, zum Okkludieren von schlauchförmigen organischen, menschlichen oder tierischen Körperteilen, wie Blutgefäße, vorzugsweise Arterien. Das chirurgische Instrument ist aus einem zweiteiligen Instrumentenschaft, einer Greifeinrichtung und einer Betätigungseinrichtung gebildet. Der Instrumentenschaft besteht aus zwei Branchen, die am distalen Ende eine Greifeinrichtung und am proximalen Ende eine Betätigungseinrichtung aufweisen, wobei die eine Branche relativ zu der anderen Branche verschiebbar ist. Die Greifeinrichtung weist zwei Greifelemente auf, wobei ein erstes Greifelement mit der verschiebbaren Branche einstückig und starr ausgebildet ist. Das zweite Greifelement ist über ein erstes Befestigungsmittel an der verschiebbaren Branche beweglich angeordnet und über ein zweites Befestigungsmittel beweglich mit der starren Branche verbunden. Die Betätigungseinrichtung besteht aus zwei Griffelementen, wobei ein erstes Griffelement mit der starren Branche einstückig und starr ausgebildet ist. Das zweite Griffelement ist an der starren Branche über ein erstes Befestigungsmittel beweglich angeordnet und über ein zweites Befestigungsmittel beweglich mit der verschiebbaren Branche verbunden und umfasst nahe den Griffelementen eine Arretierungsvorrichtung, die Rastzähne aufweisende Klinken enthält, mit der die Greifelemente in einer von mehreren Stellungen eingestellt werden können.

Allgemein bezieht sich die vorliegende Erfindung auf das Gebiet der Medizin, insbesondere der Chirurgie. Im chirurgischen Bereich werden Instrumente, Vorrichtungen oder Verfahren eingesetzt, um das Innere von lebenden Organismen zu untersuchen und/oder für operative Eingriffe zu nutzen. Zu den chirurgischen Instrumenten zählen alle medizinischen Instrumente, die vornehmlich in der Chirurgie Verwendung finden. Dazu gehören auch chirurgische Instrumente zum Abbinden oder anderweitigen Zusammendrücken von röhren-, bzw. schlauchförmigen Körperteilen, vorzugsweise von Blutgefäßen. Solche chirurgischen Instrumente werden dem Bereich der fassenden, bzw. klemmenden Instrumente zugeordnet und sind in einer großen Typenvielfalt vorhanden und hinreichend bekannt. Fassende, bzw. klemmende chirurgische Instrumente werden beispielsweise bei Eingriffen in der Herz-, Thorax- und Gefäßchirurgie verwendet. In der Herz-und Thoraxchirurgie wird meistens eine offene Operation durchgeführt, bei der durch die Eröffnung des Thorax z.B. ein Zugang zum Herzen geschaffen wird. Der Zugang erfolgt in der Regel mittels einer medianen Sternotomie, wobei ein in etwa 25 cm langer Längsschnitt durch das Brustbein erzeugt wird. Der Längsschnitt wird zum Öffnen des Brustkorbes benötigt. Bei der Thorakotomie erfolgt die chirurgische Öffnung des Thorax durch einen Interkostalschnitt, d.h., einen kleinen Schnitt in den Rippenzwischenraum. Die durch die Sternotomie oder Thorakotomie erzeugte Öffnung wird mittels eines Rippenspreizers, der zum Aufdehnen und Offenhalten des Brustkorbes eingesetzt wird, freigehalten. Die Öffnung dient den Chirurgen als Zugang für operative Eingriffe. Die Eingriffe an organischen Körperteilen erfolgen dann durch die erzeugte Öffnung im Brustkorb mit Hilfe einer Vielzahl unterschiedlicher chirurgischer Instrumente. Ist beispielsweise das Herz des Patienten freigelegt, werden direkt an das Herz und die großen Blutgefäße verschiedene Katheder, Kanülen und Klemmen angelegt. Typischerweise wird die Aorta mit einer Gefäßklemme rund um die aufsteigende Aorta okkludiert, um die Koronararterien vom Rest des Arteriensystems zu isolieren. Die notwendigerweise eingesetzten chirurgischen Instrumente verkleinern einerseits die Öffnung und behindern die Tätigkeit des Chirurgen in seinem Gesichtsfeld und andererseits ist ein schneller Heilungsprozess, aufgrund der Größe der Öffnung, des entstandenen Gewebeschadens und des operativen Traumas, bei dem Patienten nicht zu erwarten.

Medizinische Instrumente dieser Art, insbesondere fassende und klemmende chirurgische Instrumente in verschiedenen Bauarten und Ausführungen, haben sich als chirurgische Instrumente bei operativen Eingriffen vielfach bewährt und sind aus dem Stand der Technik bekannt.

### Stand der Technik

Zu der Vielzahl solcher, aus dem Stand der Technik bekannten Klemmen gehören z.B. die Kocher-, die Pean-, die Pennington-, die Foerster- und die Kowalski-, klemme, um nur einige aufzuzeigen. Alle vorgenannten Klemmen weisen aber den Nachteil auf, das sie in der Operationsöffnung liegen und diese verkleinern und/oder für die minimalinvasive Chirurgie nicht geeignet sind. Ein bekanntes Beispiel ist die Kocherklemme. Die Kocherklemme ist eine traumatische Klemme und gehört in die Klasse der zufassenden chirurgischen Instrumente. Eine solche Klemme wird vorwiegend dann eingesetzt, wenn Strukturen vornehmlich sicher gefasst, gehalten und dabei komprimiert werden müssen. Zu diesem Zweck hat die Kocherklemme geriffelte Branchen, damit das gefasste Gewebe nicht antegrad aus den Branchen rutscht. Eine solche Klemme liegt jedoch direkt in der Operationsöffnung.

Bekannt sind auch gebogene Aortenklemmen, z.B. nach Huland und Noldus, welche, um Rektumverletzungen zu vermeiden, zur Präparation zwischen Rektum und Denonvillierscher Faszie bei der Prostatektomie eingesetzt werden. Aufgrund der gebogenen Greifelemente können diese Instrumente bereits an den Rand der Operationsöffnung verlegt werden, verkleinern aber trotzdem die OP-Öffnung.

Weitere Aortenklemmen mit gebogenen Greifelementen können bei einer "Stand der Technik-Recherche" im Internet über die Suchmaschine bei Google ermittelt und als Bilder angezeigt werden, der Link lautet: https://www.google.de/search?q=aortenklemme&tbm=isch&tbo=u&source=univ&sa= X&sqi=2&ved=0ahUKEwiYvsaMs6nVAhVRKVAKHfcpB_IQsAQIJw&biw=1680&bih=8 89". Aus dem Rechercheergebnis ist die Vielzahl von Aortenklemmen ersichtlich.

Eine solche, aus der großen Typenvielfalt hergestellte chirurgische Klemme, ist der DE 20 2008 001 675 U1 zu entnehmen. Die Klemme kann, zum Instrumentenschaft hin abgewinkelte Griffelemente aufweisen, wodurch die Klemme relativ nah an den Rand einer Operationsöffnung verlegt werden kann, wobei die Griffelemente aber immer noch störend in der OP-Öffnung verbleiben. Ein weiteres Beispiel einer Klemme offenbart die DE 200 16 854 U1. Diese Klemme umfasst einen Körper mit einem Werkzeugträgerkopf und mehrere Hebel zum Bewegen des Werkzeuges, wobei die Hebel am Körper angelenkt sind und die Griffabschnitte dieser Hebel gegenüber ihrer Schwenkebene geneigt sind. Auch sind die Griffelemente sind immer noch störend.

Aus der DE 20 2007 016 057 U1 ist eine Klemme ersichtlich, die auf die störenden Griffelemente verzichtet und durch eine Betätigungseinrichtung ersetzt, welche die Griffelemente überflüssig macht und trotzdem eine Verstellung der Greifelemente ermöglicht.

Der Abklemmvorgang von Blutgefäßen durch die zuvor aufgezählten chirurgischen Instrumente erfolgt nach dem gleichen Prinzip. Die beiden Branchen der chirurgischen Instrumente werden, mit Hilfe von Ringgriffen, aufeinander zu und voneinander weg bewegt. Eine Klink- oder Sperreinrichtung, mit der die Branchen in einer von mehreren einzelnen Stellungen eingestellt werden können, ist an den Scherengliedern angeordnet. An den beiden Scherengliedern befinden sich jeweils eine Klinke und mit dieser zusammenwirkende Zähne, bzw. sind an den Scherengliedern Zahnleisten mit Rastzähnen als Arretierungseinrichtung angeordnet. Die Einstellung der Abstände der Branchen zueinander kann also nur in Stufen erfolgen. Der große Nachteil bei einer Stufeneinteilung ist, dass ein Gefäß entweder zu stark oder zu schwach abgeklemmt wird.

Als nächstliegender Stand der Technik wird der Gegenstand der DE 696 32 451 T2 angesehen. Der offenbarte Gegenstand betrifft beispielweise, nach Absatz [0034] bis [0038] und der Figur 4 im veröffentlichten Dokument, eine Zange. Die Zange weist ein manuelles Betätigungselement, eine Greifeinrichtung, die über ein zweiteiliges Körperelement funktionell mit dem Betätigungselement verbunden ist und zwei Greifelemente auf. Die Greifelemente sind so angepasst, dass sie sich als Reaktion auf eine Bewegung des Betätigungselements, öffnen oder schließen, wobei eine durch die Greifeinrichtung aufgespannte Greifebene gegen die Längsachse des Körperelements abgewinkelt ist.

Eine Gemeinsamkeit aller vorgenannten chirurgischen Instrumente ist deren Größe, die scherenartige Form mit überwiegend ringförmigen Griffen zur Betätigung der Branchen und eine typische Sperreinrichtung. Jüngste Entwicklungen in der Herz-, Thorax- und Gefäßchirurgie, welche einen Weg zur minimalinvasiven Chirurgie aufzeigen, reduzieren die Größe der Zugangsöffnung im Thorax vor allem bei der Thorakotomie, um den Gewebeschaden und das operative Trauma des Patienten zu vermindern und eine Beschleunigung des Heilungsprozesses zu erzielen. Kleine Zugangsöffnungen haben für den Chirurgen den Nachteil, dass die, in der Öffnung notwendigerweise eingesetzten chirurgischen Instrumente, das Operationsfeld, bzw. das Gesichtsfeld wesentlich verkleinern, wodurch die Operationstätigkeit erschwert wird.

Der eingeschlagene Weg in der minimal-invasiven Chirurgie kann daher nur erfüllt werden, wenn die verwendeten chirurgischen Instrumente den neuen Vorgaben der kleineren OP-Öffnungen, angepasst werden. D.h., die Ausführungsformen der chirurgischen Klemmen sollen den medizinischen Anforderungen bei den operativen Eingriffen angepasst werden. Operative Eingriffe werden, um die Belastungen des Patienten und das Risiko von Nebenwirkungen zu verringern, auch zeitlich schneller durchgeführt. Dazu werden atraumatische Instrumente benötigt deren Handhabung (Handling) den operativen Eingriff wesentlich erleichtern.

### Aufgabe der Erfindung

Der Erfindung liegt daher die Aufgabe zugrunde, ein medizinisches Instrument der eingangs genannten Art für den Einsatz in der Chirurgie zu schaffen. Das chirurgische Instrument soll die vorgenannten Nachteile und Unzulänglichkeiten der bekannten Anordnungen vermeiden und eine technische Lösung angeben. Die technische Lösung besteht in einer erweiterten Funktionalität des chirurgischen Instruments, wobei die erweiterte technische Funktionalität auf eine kostengünstige Weise herstellbar sein soll. Die technische Funktionalität besteht darin, die ringförmigen Griffelemente außerhalb des Operationsfeldes zu verbringen. Also das chirurgische Instrument soll die gleichen Eigenschaften wie die, aus dem Stand der Technik bekannten chirurgischen Klemmen aufweisen und zusätzlich ein vorteilhaftes Handling bieten, wodurch das Operationsfeld, bzw. das Gesichtsfeld des Operateurs, nicht verkleinert, sondern vergrößert wird.

### Lösung der Aufgabe

Erfindungsgemäß werden diese Probleme durch die kennzeichnenden Merkmale des Patentanspruchs 1 gelöst. Vorteilhafte Ausgestaltungen und Weiterbildungen der Erfindung ergeben sich aus den nachfolgenden Unteransprüchen und den nachstehenden Beschreibungen.

Um ein mit diesen Merkmalen der vorliegenden Erfindung ausgestattetes medizinisches Instrument, insbesondere chirurgisches Instrument, zur Verwendung bei operativen Eingriffen am menschlichen oder tierischen Körper in der Chirurgie, herzustellen, wird erfindungsgemäß vorgeschlagen, das chirurgische Instrument so zu gestalten, dass dem Chirurgen eine ergonomisch gestaltete Bedieneinrichtung zur Verfügung steht, welche die Handhabung des chirurgischen Instruments, insbesondere einer Gefäßklemme, in bekannter weise ermöglicht und das Operationsfeld nicht verkleinert. Um ein derartig ausgebildetes chirurgisches Instrument zu erhalten, mussten die zuvor genannten Aufgaben gelöst werden. Die Lösung der Aufgaben besteht darin, die bisher bekannten scherenartigen, mit ringförmigen Griffen und stufenförmigen Sperrmechanismus ausgebildeten chirurgischen Instrumente, durch das nachstehend aufgezeigt Instrument aus dem Stand der Technik zu ersetzen und mit zusätzlichen Funktionen zu versehen.

Ausgehend vom Stand der Technik wurde vorgeschlagen, als Basis die Zange aus der DE 696 32 451 T2 zu benutzen und diese weiterzuentwickeln. Die Weiterentwicklung besteht darin, das Körperelement der vorgenannten Klemme mit einem erfinderischen feststellbaren Gelenkmechanismus auszustatten, ohne die normale Funktion der Klemme einzuschränken. Ein Gelenkmechanismus ermöglicht es, einen Teil der Klemme außerhalb der OP-Öffnung zu verbringen. Ein Beispiel eines Gelenkmechanismus kann der DE 60 2004 011 298 T2 entnommen werden, wobei dieser Gelenkmechanismus nicht zum Einsatz an der Zange aus der DE 696 32 451 T2 geeignet ist und nicht die Betätigungseinrichtung aus der OP-Öffnung schwenken kann.

Die Lösung zur Verbesserung des, aus der Entwicklung entstandenen chirurgischen Instruments, findet sich in den erfinderischen Merkmalen wieder, die in den Ansprüchen aufgeführt sind.

### Beschreibung der Erfindung

Hierbei handelt es sich um ein chirurgisches Instrument zum Okkludieren von schlauchförmigen organischen Körperteilen. Das chirurgische Instrument ist aus einem zweiteiligen Instrumentenschaft, einer Greifeinrichtung, einer Betätigungseinrichtung und einem Sperrelement gebildet. Beispielsweise wird ein Sperrelement in der DE 201 12 540 U1 offenbart. Dieses Sperrelement ist aber nicht für den Einsatz am Instrumentenschaft der erfinderischen Klemme geeignet, sondern nur an scherenartigen Klemmen.

Gattungsgemäße Instrumente der vorliegenden Erfindung werden in die Gruppe der Schiebeschaftinstrumente eingeordnet. Solche Schiebeschaftinstrumente finden insbesondere im Bereich der minimalinvasiven endoskopischen Eingriffe Anwendung. In der Regel weisen sie einen dünnen, lang gestreckten Schaft auf, der einen Durchmesser zwischen 5 mm und 15 mm hat. An seinem distalen Ende ist ein Arbeitswerkzeug als Greifeinrichtung vorgesehen. Diese Greifeinrichtung weist in der Regel ein bewegbares Werkzeugelement in Form von Greifelementen auf, welche insbesondere als Zange, als Schneideinrichtung oder als fassende Klemme ausgebildet ist. Die Bewegung der Greifeinrichtung wird durch die, am proximalen Ende des Schaftes vorgesehene Betätigungseinrichtung bewirkt. In der Regel handelt es sich dabei um eine Scherengriffeinrichtung mit einer seitlich angeordneten feststehenden Branche und einer daran verschwenkbar gelagerten Griffbranche, beziehungsweise einem Griffelement.

Der Instrumentenschaft dient der Übertragung einer Betätigungsbewegung und einer Schwenkbewegung, die für eine senkrecht zur Längsachse und senkrecht zu den Achsen der Befestigungsmittel verlaufende Gelenkachse geeignet ist, wobei der Instrumentenschaft aus zwei Branchen besteht, die am distalen Ende eine Greifeinrichtung und am proximalen Ende eine Betätigungseinrichtung aufweisen, wobei die eine Branche relativ zu der anderen Branche verschiebbar ist und beide Branchen einen Gelenkmechanismus aufweisen.

Die Greifeinrichtung des chirurgischen Instruments weist zwei Greifelemente auf, wobei ein erstes Greifelement mit der verschiebbaren Branche einstückig und starr ausgebildet ist und das zweite Greifelement über ein erstes Befestigungsmittel an der verschiebbaren Branche beweglich angeordnet und über ein zweites Befestigungsmittel mit der starren Branche beweglich verbunden ist.

Die Betätigungseinrichtung des chirurgischen Instruments besteht aus zwei Griffelementen, wobei ein erstes Griffelement mit der starren Branche einstückig und starr ausgebildet ist und das zweite Griffelement an der starren Branche über ein erstes Befestigungsmittel beweglich angeordnet und über ein zweites Befestigungsmittel beweglich mit der verschiebbaren Branche verbunden ist. Die Betätigungseinrichtung umfasst nahe den Griffelementen eine Arretierungsvorrichtung, die Rastzähne aufweisende Klinken enthalten.

Es ist von besonderem Vorteil, den aus zwei Branchen bestehenden Instrumentenschaft nochmals aufzuteilen in zwei proximale- und zwei distale Branchenteile, wobei einerseits die zwei proximalen Branchenteile mit einem Ende mit den Griffelementen und andererseits die zwei distalen Branchenteile mit einem Ende mit den Greifelementen verbunden sind.

Dieser Vorteil, den Instrumentenschaft zu teilen, wird genutzt, um den Instrumentenschaft erfindungsgemäß an der Trennstelle mit einem Gelenkmechanismus auszustatten. Ein Gelenkmechanismus wird an den freien, einander zuweisenden Enden der Branchenteile, zwischen den proximalen- und distalen Branchenteilen angeordnet.

Vorteilhafterweise besteht ein Gelenkmechanismus aus einem Gelenk, wobei das Gelenk aus einer Nut und einer Feder gebildet wird. Ein solches Gelenk ist zwischen die, in proximal- und distal aufgeteilten Branchenteile der ersten Branche, angeordnet. Gleiches trifft auf die zweite Branche zu, auch hier ist ein Gelenk zwischen die, ebenfalls in zwei Branchenteile aufgeteilte Branche, angeordnet. In einer ersten Ausführung sind der proximale Branchenteil mit einer Feder und der distale Branchenteil mit einer Nut ausgebildet, wobei die Feder in die Nut eingreift und mit dieser über ein Befestigungsmittel verbunden ist, wodurch eine gelenkige Verbindung zwischen den Branchenteilen entsteht. In einer zweiten Ausführung sind die Feder und die Nut vertauscht, wodurch sich die Nut im proximalen- und die Feder im distalen Branchenteil befindet. Andere technische Gelenkverbindungen zwischen den Branchenteilen sind denkbar und daher nicht vom Schutzumfang ausgeschlossen. Mit der Integration von zusätzlichen Gelenken in den Instrumentenschaft, wird eine neue Funktion für das chirurgische Instrument ermöglicht. Die Gelenke zwischen den Branchenteilen des Instrumentenschaftes ermöglichen dem Bediener, die Betätigungseinrichtung aus der Längsachse des chirurgischen Instruments zu verschwenken. Das Verschwenken der Betätigungseinrichtung hat den Vorteil, dass die Betätigungseinrichtung bei der Operation nicht mehr in der operativen Öffnung verbleibt.

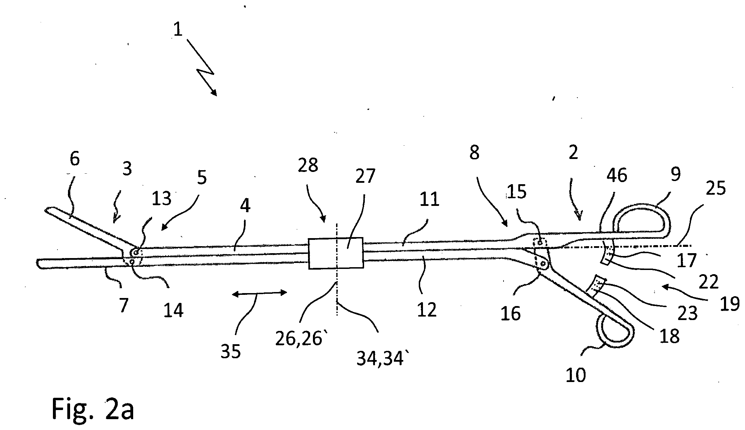

Damit das chirurgische Instrument trotz Sperrelement einsetzbar ist, wie vom Chirurgen gewohnt, muss es zuerst die normalen Funktionen als Klemme erfüllen, bevor ein Verschwenken der Betätigungseinrichtung erfolgen kann. Wenn die Betätigungseinrichtung bedient wird, verschiebt sich die eine bewegliche Branche (mit dem einen proximalen- und distalen Branchenteil und dem einem Gelenk) des Instrumentenschaftes axial gegen die andere starre Branche (mit den zwei anderen Branchenteilen und dem anderen Gelenk) des Instrumentenschaftes, wodurch die Greifeinrichtung geöffnet oder geschlossen wird. Weil das Gelenk bei der Verschiebung der beweglichen Branche aber innerhalb des Sperrelements verbleibt, besteht die ganze Zeit über ein feststehendes Gelenk, welches mit der beweglichen Brancheaxial vor- und zurück verschoben wird. Aufgrund des feststehenden Gelenks kann ein Öffnen und Schließen der Greifeinrichtung erfolgen. In der vorliegenden Ausführungsform ist das chirurgische Instrument als Klemme einfach wirkend und mit geraden Greifelementen ausgeführt. Erst durch die Betätigung der Betätigungseinrichtung beim Öffnen des chirurgischen Instruments und somit der Greifeinrichtung, verschiebt sich die bewegliche Branche in axialer Richtung zur Greifeinrichtung hin und beim Schließen des chirurgischen Instruments bzw. der Greifeinrichtung mit Hilfe der Betätigungseinrichtung, wieder axial in die Ausgangsstellung zurück. Durch die Verschiebung der beweglichen Branche in beiden axialen Richtungen, beim Öffnen und Schließen der Greifeinrichtung, verschiebt sich auch das Gelenk ebenfalls in axialer Richtung gegenüber dem Gelenk der feststehenden Branche. Die Verschiebung der beweglichen Branche in axialer Richtung erfolgt nur, wenn sich das Sperrelement in der Sperrstellung über dem Gelenkmechanismus und gleichzeitig über den beiden Branchen, wie in der Figur 2a aufgezeigt, befindet.

Erfindungsgemäß ist das chirurgische Instrument daher mit einem Sperrelement ausgebildet, welches die beweglichen Gelenke zwischen den Branchenteilen umfasst, um die Gelenke festzusetzen und somit ein Verschwenken der Branchenteile zu verhindern. Vorteilhafterweise umfasst das Sperrelement in der Sperrstellung nicht nur die zwei Gelenke, sondern auch rechts und links der Gelenke die proximalen- und distalen Branchenteile des Instrumentenschafts, wodurch ein Verschwenken der Branchenteile um das Gelenk zueinander nicht möglich ist. Trotzdem muss das Sperrelement die Funktion der chirurgischen Klemme ermöglichen. Die Funktion besteht in dem Öffnen und Schließen der Greifeinrichtung durch Bedienung der Betätigungseinrichtung. Die Bewegung der Greifeinrichtung am distalen Ende wird durch die, am proximalen Ende des Schaftes vorhandene Betätigungseinrichtung, bewirkt. Erst nachdem das chirurgische Instrument einen schlauchförmigen Körperteil erfasst und okkludiert hat, wird das Sperrelement aus der Sperrstellung verschoben.

Durch die Verschiebung des Sperrelements auf dem Instrumentenschaft aus der Sperrstellung in eine Freigabestellung, kommt der weitere Vorteil zum Einsatz. Die Verschiebung des Sperrelements erfolgt in axialer Richtung entlang der Längsachse des Instrumentenschaftes des chirurgischen Instruments. Die axiale Verschiebung des Sperrelements auf dem Instrumentenschaft kann vorteilhafterweise sowohl in Richtung der Griffelemente als auch in Richtung der Greifelemente erfolgen. In beiden Richtungen ist eine Freigabestellung für das Sperrelement auf dem Instrumentenschaft vorgesehen, in welcher die Gelenke der Branchenteile freigegeben sind. Das Sperrelement umfasst in der Freigabestellung nur noch einen distalen- oder proximalen Teil von den zwei Branchenteilen. Es umfasst nicht, wie in der Sperrstellung, über alle vier distalen- und proximalen Branchenteile, einschließlich der beiden Gelenke. Der Vorteil der Verschiebung des Sperrelements besteht darin, dass der freigegebene Gelenkmechanismus eine Schwenkbewegung der Betätigungseinrichtung ermöglicht, um die Betätigungseinrichtung mit der in einer Ebene liegenden Greifeinrichtung aus der gemeinsamen Längsachse des chirurgischen Instruments zu verschwenken. Die vorteilhafte Verschwenkung der Betätigungseinrichtung ermöglicht dem Chirurgen eine freiere Sicht auf die OP-Öffnung.

Nach erfolgter Operation kann das chirurgische Instrument wieder entfernt werden, indem die ausgeschwenkten Branchenteile mit der Betätigungseinrichtung wieder zurück auf die gemeinsame Längsachse des Instruments geschwenkt werden und das Sperrelement aus der Freigabestellung wieder in die Sperrstellung verschoben wird, wodurch das Sperrelement wieder über alle vier Branchenteile und die beiden Gelenke greift. Im Anschluss an das Sperren der Gelenke, kann über die Betätigungseinrichtung die Greifeinrichtung wieder geöffnet, dass okkludierte Körperteil wieder freigegeben und das Instrument aus der Körperöffnung entfernt werden.

Vorteilhafterweise ist das Sperrelement mit Rastmitteln versehen, welche das Sperrelement in der Freigabe- oder Sperrstellung sicher fixieren.

Ein weiterer Vorteil des erfindungsgemäßen chirurgischen Instruments besteht darin, dass das Instrument den Vorschriften des Medizinproduktgesetzes entspricht und ist, aufgrund der verwendeten Materialen, bei der Reinigung für den Einsatz zur Sterilisation geeignet.

Das erfinderische chirurgische Instrument, ausgestattet mit einem Gelenkmechanismus und einem Sperrelement für den Gelenkmechanismus, ist auf kostengünstige Weise herstellbar, weil das verschiebbare Sperrelement derart ausgebildet ist, das es die axiale Bewegung der verschiebbaren Branche zu der starren Branche nicht behindert. Die Sperrwirkung des Sperrelements auf den Gelenkmechanismus lässt sich allein durch die Anlageflächen (Innenflächen) des Sperrelements gegenüber den Anlageflächen (Außenflächen) des Instrumentenschaftes erreichen und wenn das Sperrelement im Bereich der Gelenke in beiden Richtungen über die proximalen- und distalen Branchenteile greift, also sich in der Sperrstellung befindet.

Das Sperrelement kann vorteilhafterweise aus einem Kunststoffmaterial gefertigt sein und dabei die gewünschte Funktionalität erfüllen. Auch verschieden Metalle, die für medizinische Zwecke zugelassen sind, können verwendet werden. Fertigungstechnisch günstig sind aber Kunststoffmaterialien..

### Zeichnungen

Ein Ausführungsbeispiel der Erfindung ist in den Zeichnungen rein schematisch dargestellt und wird nachfolgend näher beschrieben. Es zeigt
- Figur 1: in schematischer Darstellung eine Aortenklemme aus dem Stand der Technik, und
- Figur 2a: in schematischer Darstellung eine Ausführungsform des erfindungsgemäßen chirurgischen Instruments mit einer ersten Ausführungsform eines Sperrelements in geschlossener Stellung, und
- Figur 2b: in schematischer Darstellung das erfindungsgemäße chirurgische Instrument mit Sperrelement in geöffneter Stellung, und
- Figur 2c: in perspektivischer Darstellung das erfindungsgemäße chirurgische Instrument mit Instrumentenschaft in abgewinkelter Stellung, und
- Figur 3a-3c: in schematischer Darstellung werden verschiedene Ausführungsformen eines Sperrelements für das erfindungsgemäße chirurgische Instrument dargestellt.

### Beschreibung eines Ausführungsbeispiels

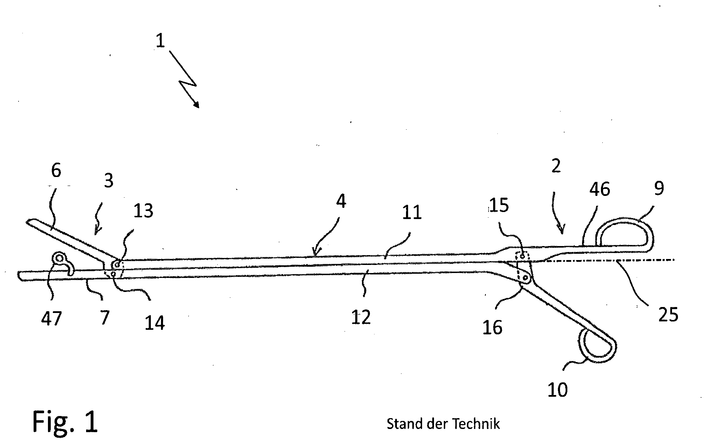

Das in der **Fig.1** in prinzipieller Darstellung aus dem Stand der Technik aufgezeigte chirurgische Instrument **1** stellt eine Zange mit Fadenführung dar und besteht im Wesentlichen aus einer Betätigungseinrichtung **2**, einem Körperelement **4** nachstehend als Instrumentenschaft **4,** bezeichnet und einer Greifeinrichtung **3**.

Die Betätigungseinrichtung **2** weist einen Schenkel **46** auf, der einstückig mit der Branche **11** des Instrumentenschafts **4** und dem Griffelement **9** ausgeführt ist, sowie ein bewegliches Griffelement **10,** das um ein Befestigungsmittel **15** (Drehzapfen) drehbar am Schenkel der Betätigungseinrichtung **2** angeordnet ist. Der Schenkel **46,** einschließlich dem starren Griffelement **9** und das bewegliche Griffelement **10** der Betätigungseinrichtung **2** sind mit einer Fingeröse versehen. Das bewegliche Griffelement **10** der Betätigungseinrichtung **2** trägt einen Stift **16,** an dem ein hinteres Ende einer weiteren Branche **12** des Instrumentenschaftes **4** drehbar montiert ist.

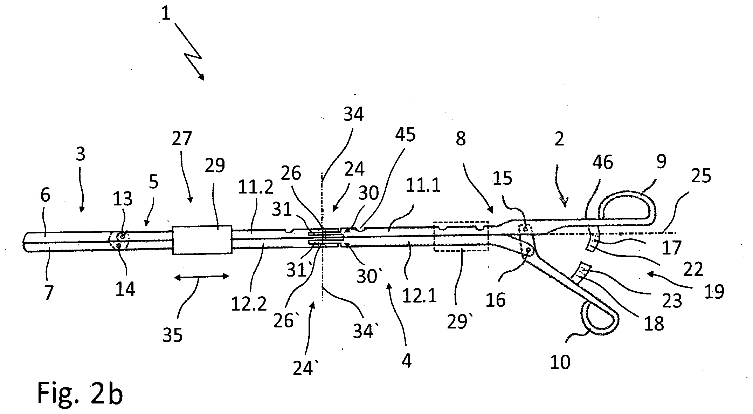

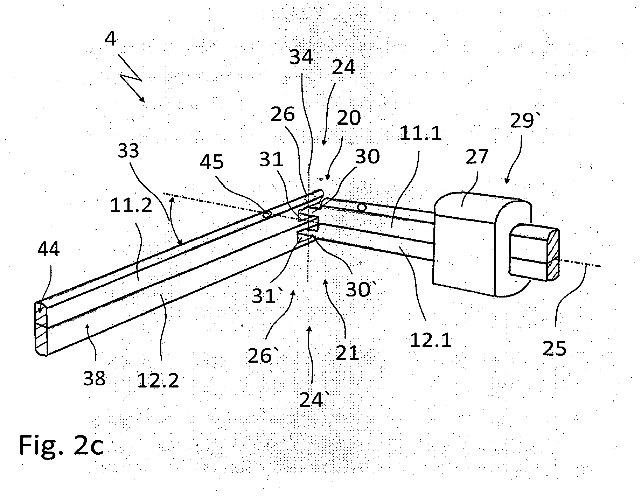

Eines der Greifelemente **7** der Greifeinrichtung **3** ist einstückig mit der Branche **12** ausgeführt. Das andere Greifelement **6** der Greifeinrichtung **3** ist an einem Ende, in der Nähe des Fußes des Greifelementes **7,** drehbar mit einem Stift **14** der Branche **12** verbunden und durch einen Stift **13,** in der Nachbarschaft des Stifts **14,** drehbar mit einem Vorderende der Branche **11** verbunden. Das chirurgische Instrument **1** führt die Öffnungs- und Schließvorgänge auf folgende Weise aus: Wenn die Betätigungseinrichtung **2** bedient wird, verschiebt sich die Branche **12** des Instrumentenschaftes **4** axial gegen die Branche **11** des Instrumentenschaftes **4.** Der Stift **14** und der Stift **13** werden dabei gegeneinander verschoben und das Greifelement **6** wird gesteuert, wodurch die Greifeinrichtung **3** geöffnet oder geschlossen wird. In dieser Ausführungsform ist das chirurgische Instrument **1** als Klemme einfach wirkend und mit gerade ausgeführten Greifelementen **6, 7** ausgeführt. Die freien Endabschnitte des Greifelements **7** und des Greifelements **6** können jeweils in einem Winkel zur Längsachse **25** des Instrumentenschaftes **4** gebogen (nicht dargestellt) sein, und die Fadenführung **47** kann innerhalb der durch die gebogenen Abschnitte aufgespannten Greifebene angeordnet sein. Aufgrund einer anderen Verwendung des erfinderischen chirurgischen Instruments **1,** wie in der in **Figur 2** aufgezeigt, entfällt bei der Klemme die Fadenführung **47.**

In der **Fig.2a** ist, in schematischer Darstellung, ein erfindungsgemäßes chirurgisches Instrument 1 mit Sperrelement **27** in der Sperrstellung **28,** aufgezeigt. Die in der **Figur 1** aufgezeigten Bezugszeichen werden hier analog übernommen. Auch die Beschreibung des chirurgischen Instruments **1** aus der **Figur 1****,** kann für das, in der **Figur 2** aufgezeigte erfinderische chirurgische Instrument **1** teilweise übernommen werden, weil die Basis des chirurgischen Instruments **1,** wie in der **Figur 1** aufgezeigt, in der Ausführungsform dem chirurgischen Instrument **1** in der **Figur 2** entspricht. Die Ausführungsform weist einen langen Instrumentenschaft **4** auf, der sich von den scherenartigen Klemmen aus dem Stand der Technik unterscheidet, bei der die Schenkel vom Griffstück bis zur Klemmbranche durchgängig und einstückig ausgebildet sind und sich im Kreuzungsbereich mit einem gemeinsamen Schwenklager überlappen. Die Länge des Instrumentenschaftes **4** ist für den Einsatz des Instrumentes **1** in der minimal-invasiven Chirurgie ausgelegt. Solche transthorakalen Klemmen werden bei Operationen unter endoskopischer Sicht verwendet. Diese Instrumente werden beispielsweise bei kleinen seitlichen Zugangsöffnungen im Rippenzwischenraum eines Brustkorbes eingesetzt, z.B. im rechten Thoraxbereich, um Zugang zur Aorta im linken Brustraum zu erhalten. Aktuell wird in den meisten OP-Zentren eine solche transthorakale Aortenklemme, die sog. Chitwood-Klemme (z. B. Scanlan International, Fa. St. Paul, Minn.) verwendet. Diese wird durch eine Stichinzision im ca. 3. ICR kranial der Thorakotomie in den Thorax eingebracht und zum externen Abklemmen der Aorta ascendens verwendet. Die Klemme wird unter Sicht durch den Sinus transversus eingebracht. Hierbei wird auf das linke Vorhofohr geachtet, um es nicht zu verletzten. Zum Abklemmen der Aorta wird die HLM kurz angehalten, so dass die Klemmung kontrolliert und ohne Hebel erfolgen kann.

Das erfinderische chirurgische Instrument **1** ist aus einem langen zweiteiligen Instrumentenschaft **4,** einer Greifeinrichtung **3,** einer Betätigungseinrichtung **2** sowie einem Sperrelement **27** gebildet und ist im geöffneten Zustand der Greifeinrichtung **3** und mit nicht arretierter Betätigungseinrichtung **2** dargestellt.

Der Instrumentenschaft **4** sorgt für die Übertragung einer Betätigungsbewegung, ausgehend von den Griffelementen **9, 10** über die Branchen **11, 12** des Instrumentenschaftes **4** bis zu den Greifelementen **6, 7** und sorgt auch für die Schwenkbewegung der Betätigungseinrichtung **2** um eine Gelenkachse **26, 26',** die senkrecht zur Längsachse **25** und senkrecht zu den Achsen der Befestigungsmittel **13, 14, 15, 16** angeordnet ist. Des Weiteren besteht der Instrumentenschaft **4** aus zwei Branchen **11, 12,** die am distalen Ende **5** eine Greifeinrichtung **3** und am proximalen Ende **8** eine Betätigungseinrichtung **2** aufweisen, wobei die eine Branche **12** relativ zu der anderen Branche **11** verschiebbar ist und beide Branchen **11, 12** einen Gelenkmechanismus **24, 24'** mit einer Gelenkachse **26, 26'**, siehe **Figur 2b** aufweisen. Aus der **Figur 2a** ist weiterhin ersichtlich, dass das Sperrelement **27** sich in einer arretierten Sperrstellung **28** befindet. In der Sperrstellung **28** sowie im geschlossenen Zustand der Greifeinrichtung **3** und mit arretierter Betätigungseinrichtung **2,** befinden sich die Gelenke **20, 21** des Gelenkmechanismus **24, 24'** bzw. deren beiden Gelenkachsen **26, 26'** auf einer gemeinsamen Mittellinie **34,** siehe **Figur 2b****.** Erst durch die Betätigung der Betätigungseinrichtung **2** beim Öffnen des chirurgischen Instruments **1** und somit der Greifeinrichtung **3,** verschiebt sich die bewegliche Branche **12** in axialer Richtung **35** zur Greifeinrichtung **3** hin und beim Schließen des chirurgischen Instruments **1** bzw. der Greifeinrichtung **3,** mit Hilfe der Betätigungseinrichtung **2,** wieder axial in die Ausgangsstellung zurück. Durch die Verschiebung der Branche **12** in beide axialen Richtungen **35,** beim Öffnen und Schließen der Greifeinrichtung **3,** verschiebt sich auch die Gelenkachse **26'** ebenfalls in axialer Richtung **35** gegenüber der Gelenkachse **26** der feststehenden Branche **11.** Die Verschiebung der beweglichen Branche **12** in axialer Richtung **35** erfolgt nur, wenn sich das Sperrelement **27** in der Sperrstellung **28** über dem Gelenkmechanismus **24, 24'** und gleichzeitig über den beiden Branchen **11, 12,** wie in der **Figur 2a** aufgezeigt, befindet. Das Gelenk **21** der Branche **12** ist somit nur in axialer Richtung **35** der, am distalen Ende **5** befindlichen Greifeinrichtung **3**,hin und in die Ausgangsposition axial zurück verschiebbar. In der Ausgangsposition ist die Mittellinie **34'** des Gelenks **21** deckungsgleich mit der Mittellinie **34** des Gelenks **20.** In der axial verschobenen Position des Gelenks **21** der Branche **12** ist die Mittellinie **34'** des Gelenks **21** ebenfalls gegenüber der Mittellinie **34** des Gelenks **20** verschoben. Der axiale Verschiebeweg der Branche **12** und somit des Gelenks **21** ergibt sich aus dem Winkelbereich, der vom radial schwenkbaren Griffelement **10** der Betätigungseinrichtung **2** auf die axial verschiebbare Branche **12** übertragbar ist. Die Greifeinrichtung **3** weist zwei Greifelemente **6, 7** auf, wobei ein erstes Greifelement **7** mit der verschiebbaren Branche **12** einstückig und starr ausgebildet ist und das zweite Greifelement **6** über ein erstes Befestigungsmittel **14** an der verschiebbaren Branche **12** beweglich angeordnet ist und über ein zweites Befestigungsmittel **13** mit der starren Branche **11** beweglich verbunden ist.

Die Betätigungseinrichtung **2** hingegen besteht aus zwei Griffelementen **9, 10,** wobei ein erstes Griffelement **9** mit der starren Branche **11** einstückig und starr ausgebildet ist und das zweite Griffelement **10** an der starren Branche **11** über ein erstes Befestigungsmittel **15** beweglich angeordnet ist und über ein zweites Befestigungsmittel **16** beweglich mit der verschiebbaren Branche **12** verbunden ist. Die Betätigungseinrichtung **2** umfasst nahe den Griffelementen **9, 10** eine Arretierungsvorrichtung **19,** die Rastzähne **22, 23** aufweisende Klinken **17, 18** enthält. Aus der **Figur 2a** ist weiterhin ersichtlich, dass die Betätigungseinrichtung **2** sich nicht in einer arretierten Stellung befindet. In einer Arretierungsstellung wirken die Rastzähne **22, 23** der Klinken **17, 18** zusammen und die Greifelemente **6, 7** der Greifeinrichtung **3** umfassen ein Körperteil oder, wie in der vorliegenden Darstellung, die Greifelemente **6, 7** befinden sich in der geöffneten Stellung. In der geschlossenen Stellung der Greifelemente **6, 7** des chirurgischen Instruments **1** liegen die Klinken **17, 18** übereinander und die Rastzähne **22, 23** greifen ineinander. Das Sperrelement **27** befindet sich in der arretierten Sperrstellung **28** über den Gelenken **20, 21** der Branchen **11, 12** des Instrumentenschaftes **4,** wodurch ein Verschwenken der Betätigungseinrichtung **2** aus der Längsachse **25** gegenüber der Greifeinrichtung **3** nicht möglich ist. Trotzdem ist in der Sperrstellung **28** des Sperrelements **27** ein Öffnen und Schließen der Greifeinrichtung **3** durch ein Verschieben der Branche **12** mit Hilfe der Betätigungseinrichtung **2** möglich.

In der **Fig.2b** ist in schematischer Darstellung ein erfindungsgemäßes chirurgisches Instrument **1** mit Sperrelement **27** in der Freigabestellung **29, 29'** aufgezeigt. Die, in der **Figur 1** und **Figur 2a** aufgezeigten Bezugszeichen werden hier analog übernommen. Auch die Beschreibung des chirurgischen Instruments **1** aus der **Figur 1** **und** **Figur 2a** wird für das, in der **Figur 2b** aufgezeigte erfinderische chirurgische Instrument **1,** zum Teil übernommen.

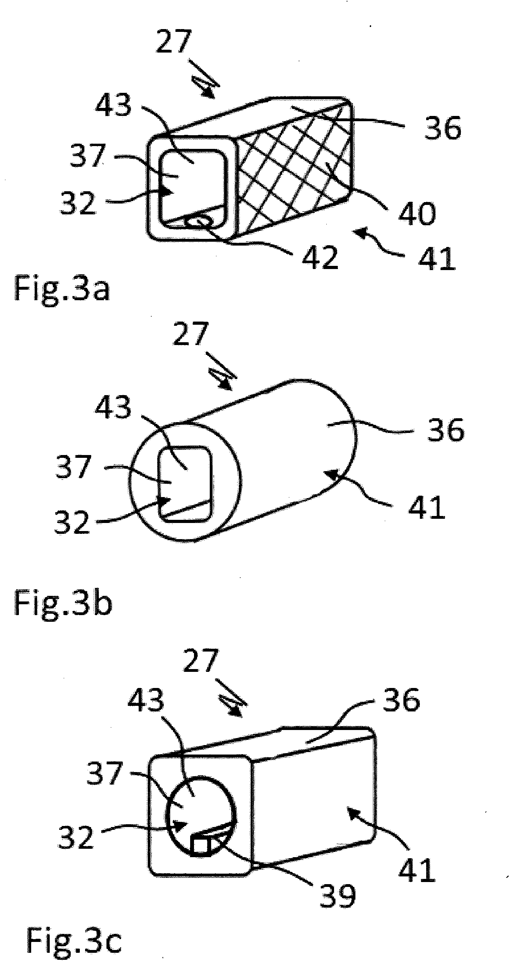

Das, sich in einer arretierten ersten Freigabestellung **29** auf dem Instrumentenschaft **4** befindliche Sperrelement **27,** gibt die Gelenke **20, 21** des Gelenkmechanismus **24, 24'** des, aus Branchen **11, 12** bestehenden Instrumentenschaftes **4,** frei. Die Freigabe der Gelenke **20, 21** erfolgt durch Verschiebung des Sperrelements **27** aus der Sperrstellung **28** in axialer Richtung **35** bis zu einer Freigabestellung **29, 29'**. Die axiale Verschiebung des Sperrelements **27** auf dem Instrumentenschaft **4,** kann sowohl in Richtung des distalen Endes **5** als auch in Richtung des proximalen Endes **8** des chirurgischen Instruments **1** erfolgen. Die axiale Verschiebung des Sperrelements **27** in beiden Richtungen erfolgt nur so weit, bis die beiden Gelenke **20, 21** freigegeben sind. Nach der Freigabe der Gelenke **20, 21** befindet sich das Sperrelement **27** in der Freigabestellung **29.** Es sind zwei Freigabestellungen **29, 29'** für das Sperrelement **27** vorgesehen. Die erste Freigabestellung **29** des Sperrelements **27** befindet sich im distalen Branchenteil **11.2, 12.2,** während die zweite Freigabestellung **29'** im proximalen Branchenteil **11.1, 12.1** angeordnet ist. Die Sperrstellung **28** sowie die beiden Freigabestellungen **29, 29'** für das Sperrelement **27,** können mit Rastelementen **45** auf oder in dem Instrumentenschaft **4** versehen sein, um den axialen Schiebeweg des Sperrelements **27** zu begrenzen. Auch das Sperrelement **27** kann mit Rastmitteln **42,** siehe **Figur 3a****,** ausgebildet sein, wobei diese Rastmittel **42** des Sperrelements **27** mit den Rastmitteln **45** des Instrumentenschaftes **4** korrespondieren. Aufgrund der Rastmittel **42, 45,** wird das Sperrelement **27** in der Sperrstellung **28** oder der Freigabestellung **29, 29'** fixiert.

Die beiden Branchen **11, 12** des Instrumentenschafts **4** sind jeweils aus einem proximalen- und distalen Branchenteil **11.1, 11.2, 12.1, 12.2** gebildet, wobei das eine Ende **8** des proximalen Branchenteil **11.1, 12.1** mit dem Griffelement **9, 10** und das eine Ende **5** des distalen Branchenteil **11.2, 12.2** mit dem Greifelement **6, 7** verbunden ist. Am anderen distalen- und proximalen Ende der Branchenteile **11.1, 11.2, 12.1, 12.2** befindet sich ein Gelenkmechanismus **24, 24'**. Das bedeutet, dass zwischen den distalen- und proximalen Branchenteilen **11.1, 11.2, 12.1, 12.2** ein Gelenkmechanismus **24, 24'** angeordnet ist. D.h. jede Branche **11, 12** weist einen eigenen Gelenkmechanismus **24, 24'** auf. Beide Gelenkmechanismen **24, 24'** sind von einem einzigen Sperrelement **27** umfasst. Nach dem Verschieben des Sperrelements **27** auf dem Instrumentenschaft **4** aus der Sperrstellung **28** in die Freigabestellung **29,** ist der Gelenkmechanismus **24, 24'** freigegeben. Nach der Freigabe der Gelenkmechanismen **24, 24'** wird eine Schwenkbewegung der Betätigungseinrichtung **2** ohne Betätigung der Griffelemente **9, 10** ermöglicht. Die Betätigungseinrichtung **2** kann mit der in einer Ebene liegenden Greifeinrichtung **3** somit aus der gemeinsamen Längsachse **25** gelenkig, siehe **Figur 2c****,** verschwenkt werden, ohne das die Greifelemente **6, 7** eine Ortsveränderung erfahren.

Jeder Gelenkmechanismus **24, 24'** umfasst ein Gelenk **20, 21,** welches auf der proximalen- oder distalen Seite des Branchenteils **11.1, 11.2, 12.1, 12.2** eine Feder **30, 30'** und auf der, der Feder **30, 30'** zugewandten gegenüberliegenden distalen- oder proximalen Seite des Branchenteils **11.1, 11.2, 12.1, 12.2,** eine korrespondierende Nut **31, 31'** aufweist. Die Feder **30, 30'** und die Nut **31, 31'** greifen ineinander und sind über ein Befestigungsmittel, bestehend aus einer Gelenkachse **26, 26',** verbunden. D.h., die Feder **30, 30'** kann sowohl am Branchenteil **11.1** und **12.1** und die Nut **31, 31'** am Branchenteil **11.2** und **12.2,** wie aus der **Figur 2b** ersichtlich, angeordnet sein. Natürlich können die Nut **30, 30'** und die Feder **31, 31'** auch entgegengesetzt angeordnet sein, dann würde sich die Feder **30, 30'** am Branchenteil **11.2** und **12.2** und die Nut **31, 31'** am Branchenteil **11.1** und **12.1** befinden. Des Weiteren sind noch andere Gelenkmechanismen zum Abwinkeln des Instrumentenschaftes **4** und Verschwenkens der Betätigungseinrichtung **2** aus der gemeinsamen Längsachse **25** des chirurgischen Instruments **1** denkbar und der Gelenkmechanismus bleibt somit nicht auf die vorgenannte Ausführungsform beschränkt.

Das Sperrelement **27** weist eine Öffnung **32,** siehe **Figur 3a****,** auf, die mit der Form des Instrumentenschaftes **4** korrespondiert und die eine Verschiebung der verschiebbaren Branche **12** während der Sperrstellung **28** des Gelenks **20, 21,** siehe **Figur 2a****,** durch das Sperrelement **27** in Richtung der Längsachse **25** ermöglicht.

Aus der **Fig.2c** ist in schematischer Darstellung ein Instrumentenschaft **4** des chirurgischen Instruments **1,** gemäß der **Figur 2a** und **Figur 2b****,** in abgewinkelter Stellung ersichtlich. Die, in der **Figur 2a****,** **2b** aufgezeigten Bezugszeichen, werden hier analog übernommen, aber nicht näher beschrieben.

Die proximalen Branchenteile **11.1, 12.1** der Branchen **11, 12** des Instrumentenschaftes **4** sind gegenüber den distalen Branchenteilen **11.2, 12.2** um ca. 60 Grad aus der Längsachse **25** abgewinkelt und damit ist die nicht dargestellte Betätigungseinrichtung **2** um 60 Grad zur Greifeinrichtung **3** abgewinkelt. Der einstellbare Winkel **33** der um das Gelenk **20, 21** verschwenkbaren Betätigungseinrichtung **2,** kann zwischen 0 Grad und 120 Grad betragen, vorteilhafterweise kann der Winkel **33** zwischen 0 Grad und 90 Grad stufenlos eingestellt werden. Die Feder **30, 30'** des Gelenks **20, 21** dient nicht nur der drehbaren Befestigung der Branchenteile **11.1, 12.1** in der Nut **31, 31'** der Branchenteile **11.2, 12.1,** sondern dem Gelenkmechanismus **24, 24'** auch als maximaler Winkelanschlag. Die Verbindung zwischen der Feder **30, 30'** und der Nut **31, 31'** erfolgt über die Gelenkachse **26, 26'** und ist technisch derart gestaltet, das eine Verdrehung um die Gelenkachse **26, 26'** bzw. ein Verschwenken der proximalen Branchen **11.1, 12.1** des Instrumentenschaftes **4** um das Gelenk **20, 21** relativ leicht vonstattengeht, damit auf die Greifeinrichtung **3** und somit auf das okkludierte schlauchförmige Körperteil so gut wie keine Kräfte wirken. Der Gelenkmechanismus **24, 24'** ist selbsthemmend konzipiert, so dass ein Drehung bzw. ein Verschwenken der Branchenteile **11.1, 12.1** oder Branchenteile **11.2, 12.2** zueinander oder voneinander weg um das Gelenk **20, 21,** nur mit Hilfe des Handlings des Chirurgen erfolgen kann. Das Sperrelement **27** befindet sich in der Freigabestellung **29'** im proximalen Bereich der Branchenteile **11.1, 12.1** des Instrumentenschaftes **4.** Die im Instrumentenschaft 4 angeordneten Rastmittel **45** im Bereich des Gelenkmechanismus **24** sind sichtbar, weil das Sperrelement **27** sich nicht in der Sperrstellung **28,** sonder in der Freigabestellung **29'** befindet.

Die **Fig.3a, 3b, 3c** zeigen in schematischer Darstellung eine erste Ausführungsform eines Sperrelements **27** des erfindungsgemäßen chirurgischen Instruments **1.** Das chirurgische Instrument **1** weist zur Lösung der Aufgabe zwei erfinderische technische Elemente auf, mit denen neue Funktionen des chirurgischen Instruments **1** ermöglicht werden. Die erste erfinderische Lösung besteht in der Anordnung eines Gelenkmechanismus **24, 24'** im Instrumentenschaft 4, siehe **Figur 2b****,** **2c****.** Die zweite Lösung besteht in der Ausgestaltung und Funktion eines Sperrelements **27** zur Fixierung oder Freigabe der Gelenke **20, 21** des Instrumentenschaftes **4,** siehe ebenfalls **Figur 2b****,** **2c****.** Die Funktion des Sperrelements **27** besteht einerseits darin, wenn es sich in der Sperrstellung **28** befindet, die Gelenke **20, 21** des Instrumentenschaftes **4** an einer Drehbewegung zu hindern und somit die Gelenke **20, 21** festzusetzen, um eine normale Greif- und Schließtätigkeit mit dem chirurgischen Instrument **1** ausführen zu können. Andererseits besteht die Funktion des Sperrelements **27** in der Freigabe der Gelenke **20, 21,** wenn sich das Sperrelement **27** in der Freigabestellung **29, 29'** befindet, um ein Verschwenken der Betätigungseinrichtung **2** um die Gelenke **20, 21** zu ermöglichen, wobei die eingestellten Greifelemente **6, 7** der Greifeinrichtung **3** in ihrer verbrachten Stellung verbleiben und ein okkludiertes Körperelement weiterhin festhalten.

Durch das Sperrelement **27** lässt sich die Schwenkbewegung der beiden Branchenteile **11.1, 12.1** gegenüber den Branchenteilen **11.2, 12.2** sperren. Die Sperrstellung **28** ist dann gegeben, wenn das Sperrelement **27** sich direkt über dem Gelenkmechanismus **24, 24',** siehe **Figur 2a****,** befindet und gleichzeitig über die proximalen Branchenteile **11.1, 12.1** sowie über die distalen Branchenteile **11.2, 12.2** greift. Die Sperrung der Gelenke **20, 21** erfolgt in Sperrstellung **28** durch das Anliegen der Innenflächen **37** des Sperrelements **27** an den Außenflächen **38** des Instrumentenschaftes **4.**

Grundsätzlich handelt es sich bei dem Sperrelement **27,** wie aus der **Figur 3a** ersichtlich, annähernd um eine Hülse **36,** die in einer ersten Ausführungsform kastenförmig ausgebildet ist. Die Hülse **36** weist eine Öffnung **32** auf, die annähernd einer rechteckigen Durchgangsbohrung entspricht, siehe **Figur 3a****.** Die rechteckige innere Form **43** (Innenkontur) der Durchgangsbohrung korrespondiert mit der rechteckigen äußeren Form **44** (Außenkontur) des Instrumentenschaftes **4,** wobei die Innenflächen **37** der Durchgangsbohrung immer an den Außenflächen **38** des Instrumentenschaftes **4** anliegen, auch in der Freigabestellung **29, 29'** oder während der axialen Verschiebung des Sperrelements **27** entlang der Branchen **11, 12.** Die Form **44** der Branchen **11**, **12** des Instrumentenschaftes **4** bestimmen also die Form **43** der Durchgangsbohrung in der Hülse **36** des Sperrelementes **27.** Ist der Instrumentenschaft **4** in etwa rund ausgeführt, wobei jede Branche **11, 12** halbkreisförmig ausgebildet ist, ist die Öffnung **32** als Innenkontur **43** der Hülse **36** ebenfalls rund, siehe **Figur 3c****.** Andere Innenkonturen **43** bei den Hülsen **36** sind denkbar und abhängig von den äußeren Form **44** (Außenkontur) der Instrumentenschäfte **4.** Egal welche Form **44** der Instrumentenschaft **4** aufweist, die Innenflächen **37** der Hülse **36** dienen immer als Anlagefläche für die Außenflächen **38** der Branchen **11, 12,** und die Schenkel der Branchen **11, 12** dienen der Hülse **36** als Verschiebeführung. Die relative Länge der Hülse **36** ist derart ausgelegt, dass die Hülse **36** in Sperrstellung **28** noch rechts und links der Gelenke **20, 21** über die proximalen- und distalen Branchenteile **11.1, 12.1, 11.2, 12.2** reicht, um ein Verschwenken der Betätigungseinrichtung **2** zu vermeiden. Auch die Form der Außenkontur **41** der Hülse **36** kann unterschiedlich gestaltet sein. Z.B. wie in der **Figur 3a** aufgezeigt, kann die Außenkontur **41** einem Instrumentenschaft **4** nachgebildet sein oder wie in der **Figur 3c** dargestellt, eine quadratische Außenkontur **41** aufweisen oder wie der **Figur 3b** zu entnehmen, aus einer kreisförmigen Außenkontur **41** gebildet sein. Die Außenkontur **41** einer Hülse **36** kann wiederum ein erhabenes Oberflächenprofil **40** oder Vertiefungen aufweisen, um das Handling für den Chirurgen zu verbessern. Beispielsweise kann das Oberflächenprofil **40** der Hülse **36** geriffelt sein, so dass ein Bediener das Sperrelement **27** axial auf dem Instrumentenschaft **4** gut ergreifen und verschieben kann.

Des Weiteren kann die Hülse **36** des Sperrelements **27** mit Rastmitteln **42** ausgestattet sein, siehe **Figur 3a****,** wobei diese Rastmittel **42** des Sperrelements **27** mit den Rastmitteln **45** des Instrumentenschaftes **4** korrespondieren. Aufgrund der Rastmittel **42,** wird das Sperrelement **27** in der Sperrstellung **28** oder der Freigabestellung **29, 29'** fixiert. Beispielsweise können die Rastmittel **42** der Hülse **36** aus einer oder mehreren Erhöhungen, z.B. Nocken bestehen, die in der Öffnung **32** der Hülse **36** angeordnet sind. Die Nocken können z.B. am Anfang und am Ende der Öffnung **32** der Hülse **36** angeordnet sein. Ein Instrumentenschaft **4** weist eine oder mehrere entsprechende Vertiefungen als korrespondierende Rastmittel **45,** siehe **Figur 2b** auf, in welche eine der Nocken der Hülse **36** korrespondierend eingreifen kann. Vorteilhafterweise sind mehrere Vertiefungen im Instrumentenschaft **4** angeordnet uzw. im Bereich der Sperrstellung **28** des Sperrelements **27** und im Bereich der Freigabestellung **29, 29'**, um das Sperrelement **27** zu fixieren. Des Weiteren kann in der Durchgangsbohrung eine Passfeder **39** angeordnet sein, die in eine korrespondierende Nut im Instrumentenschaft **4** (nicht dargestellt) eingreifen kann. Natürlich sind weitere Rastmittel in der Hülse **36** zum fixieren des Sperrelements **27** und am Instrumentenschaft **4** denkbar und nicht auf die angegebenen Rastmittel **42, 45** beschränkt. Beispielsweise kann die Hülse **36** des Sperrelements **27** Vertiefungen und der Instrumentenschaft **4** Erhöhungen als Rastmittel **42, 45** aufweisen.

**Bezugszeichenliste**

| | | | |
|---|---|---|---|
| 1 | Chirurgisches Instrument | 31, 31' | Nut |
| 2 | Betätigungseinrichtung | 32 | Öffnung |
| 3 | Greifeinrichtung | 33 | Winkel |
| 4 | Instrumentenschaft | 34, 34' | Mittellinie |
| 5 | Distales Ende | 35 | Axiale Richtung |
| 6 | Greifelement (beweglich) | 36 | Hülse |
| 7 | Greifelement (starr) | 37 | Innenfläche |
| 8 | Proximales Ende | 38 | Außenfläche |
| 9 | Griffelement (starr) | 39 | Passfeder |
| 10 | Griffelement (beweglich) | 40 | Oberflächenprofil |
| 11 | Branche (starr) | 41 | Außenkontur (v.36) |
| 12 | Branche (verschiebbar) | 42 | Rastmittel (v.36) |
| 13 | Befestigungsmittel | 43 | Innenkontur (v.36) |
| 14 | Befestigungsmittel | 44 | Außenkontur (v.4) |
| 15 | Befestigungsmittel | 45 | Rastmittel (v.4) |
| 16 | Befestigungsmittel | 46 | Schenkel |
| 17 | Klinke | 47 | Fadenführung |
| 18 | Klinke | | |
| 19 | Arretierungsvorrichtung | | |
| 20 | Gelenk | | |
| 21 | Gelenk | | |
| 22 | Rastzähne | | |
| 23 | Rastzähne | | |
| 24, 24' | Gelenkmechanismus | | |
| 25 | Längsachse (v. 1) | | |
| 26, 26' | Gelenksachse (v.20,21) | | |
| 27 | Sperrelement | | |
| 28 | Sperrstellung | | |
| 29, 29' | Freigabestellung | | |
| 30, 30' | Feder | | |

## Patentansprüche

1. Chirurgisches Instrument **(1),** zum Okkludieren von schlauchförmigen organischen Körperteilen, bestehend aus einem zweiteiligen Instrumentenschaft **(4),** einer Greifeinrichtung **(3),** einer Betätigungseinrichtung **(2),** wobei die Betätigungseinrichtung **(2)** aus zwei Griffelementen **(9, 10)** besteht und das erste Griffelement **(9)** mit der starren Branche **(11)** einstückig und starr ausgebildet ist, und das zweite Griffelement **(10)** an der starren Branche **(11)** über ein erstes Befestigungsmittel **(15)** beweglich angeordnet und über ein zweites Befestigungsmittel **(16)** beweglich mit der verschiebbaren Branche **(12)** verbunden ist und die Betätigungseinrichtung **(2)** umfasst nahe den Griffelementen **(9, 10)** eine Arretierungsvorrichtung **(19),** die Rastzähne **(22, 23)** aufweisende Klinken **(17, 18)** enthält und wobei die Greifeinrichtung **(3)** zwei Greifelemente **(6, 7)** aufweist, wobei ein erstes Greifelement **(7)** mit der verschiebbaren Branche **(12)** einstückig und starr ausgebildet ist und das zweite Greifelement **(6)** über ein erstes Befestigungsmittel **(14)** an der verschiebbaren Branche **(12)** beweglich angeordnet und über ein zweites Befestigungsmittel **(13)** mit der starren Branche **(11)** beweglich verbunden ist, **dadurch gekennzeichnet, dass** der Instrumentenschaft **(4),** welcher zur Übertragung einer Betätigungsbewegung und einer Schwenkbewegung, die um eine senkrecht zur Längsachse **(25)** und senkrecht zu den Achsen der Befestigungsmittel **(13, 14, 15, 16)** verlaufende Gelenkachse **(26)** geeignet ist, aus zwei Branchen **(11, 12)** besteht, die am distalen Ende **(5)** eine Greifeinrichtung **(3)** und am proximalen Ende **(8)** eine Betätigungseinrichtung **(2)** aufweisen, wobei die eine Branche **(12)** relativ zu der anderen Branche **(11)** verschiebbar ist und beide Branchen **(11, 12)** einen Gelenkmechanismus **(24, 24')** aufweisen, der von einem Sperrelement **(27),** welches als Hülse **(36)** ausgebildet ist, umfasst ist.

2. Chirurgisches Instrument **(1)** nach Anspruch 1, **dadurch gekennzeichnet, dass** die beiden Branchen **(11, 12)** des Instrumentenschafts **(4)** jeweils aus einem proximalen- und distalen Branchenteil **(11.1, 11.2, 12.1, 12.2)** gebildet sind, wobei die beiden proximalen Branchenteile **(11.1, 12.1)** mit einem Griffelement **(9, 10)** und die beiden distalen Branchenteile **(11.2, 12.2)** mit einem Greifelement **(6, 7)** verbunden sind.

3. Chirurgisches Instrument **(1)** nach Anspruch 2, **dadurch gekennzeichnet, dass** zwischen den Branchenteilen **(11.1, 11.2, 12.1, 12.2)** jeweils ein Gelenkmechanismus **(24, 24'**) angeordnet ist, welcher von einem Sperrelement **(27)** umfasst ist, wobei nach dem Verschieben des Sperrelements **(27)** aus der Sperrstellung **(28)** in eine Freigabestellung **(29, 29')** eine Schwenkbewegung der Betätigungseinrichtung **(2)** ermöglicht wird, um die Betätigungseinrichtung **(2)** mit der in einer Ebene liegenden Greifeinrichtung **(3)** aus der gemeinsamen Längsachse **(25)** gelenkig zu verschwenken.

4. Chirurgisches Instrument **(1)** nach Anspruch 3, **dadurch gekennzeichnet, dass** der Gelenkmechanismus **(24, 24')** ein Gelenk **(20, 21)** umfasst, welches auf der proximalen- oder distalen Seite des Branchenteils **(11.1, 11.2, 12.1, 12.2)** eine Feder **(30, 30')** und auf der gegenüberliegenden distalen- oder proximalen Seite des Branchenteils **(11.1, 11.2, 12.1, 12.2)** eine korrespondierende Nut **(31, 31')** aufweist, welche ineinander greifen und über ein Befestigungsmittel **(26, 26')** verbunden sind.

5. Chirurgisches Instrument **(1)** nach Anspruch 3, **dadurch gekennzeichnet, dass** das Sperrelement **(27)** eine Hülse **(36)** bildet, die eine Öffnung **(32)** aufweist, wobei die Öffnung **(32)** der Hülse **(36)** mit der äußeren Form **(44)** des Instrumentenschaftes **(4)** korrespondiert und die eine Verschiebung der verschiebbaren Branche **(12),** in Sperrstellung **(28)** des Gelenks **(20, 21)** durch das Sperrelement **(27)** hindurch, in beide Richtungen der Längsachse **(25)** ermöglicht.

6. Chirurgisches Instrument **(1)** nach Anspruch 5, **dadurch gekennzeichnet, dass** die Öffnung **(32)** der Hülse **(36)** Rastmittel **(42)** aufweist, die mit Rastmitteln **(45)** des Instrumentenschaftes **(4)** in der Sperr- **(28)** und Freigabestellung **(29, 29')** des Sperrelements **(27)** korrespondieren.

7. Chirurgisches Instrument **(1)** nach Anspruch 6, **dadurch gekennzeichnet, dass** die Rastmittel **(42)** in der Hülse **(36)** Erhöhungen und die Rastmittel **(45)** im Instrumentenschaft **(4)** Vertiefungen bilden oder die Hülse **(36)** weist Vertiefungen und der Instrumentenschaft **(4)** Erhöhungen auf.

8. Chirurgisches Instrument **(1)** nach Anspruch 5, **dadurch gekennzeichnet, dass** die Hülse **(36)** aus Kunststoffmaterial gefertigt ist.

## Claims

1. A surgical instrument **(1),** for occluding tubular organic body parts, the surgical instrument consisting of a two-part instrument shaft **(4),** a gripper device **(3),** an actuation device **(2),** the actuation device **(2)** consisting of two hand grip elements **(9, 10)** and the first hand grip element **(9)** being formed in one piece with the rigid branch **(11)** and fixed, and the second hand grip element **(10)** being movably arranged on the rigid branch **(11)** via a first fastening means **(15)** and being movably connected to the displaceable branch **(12)** via a second fastening means **(16),** and the actuation device **(2)** comprising a locking apparatus **(19)** close to the hand grip elements **(9, 10),** which locking apparatus includes pawls **(17, 18)** that have detent teeth **(22, 23),** and the gripper device **(3)** having two gripper elements **(6, 7),** with a first gripper element **(7)** being formed in one piece with the displaceable branch **(12)** and fixedly, and the second gripper element **(6)** being arranged movably on the displaceable branch **(12)** via a first fastening means **(14)** and being connected movably to the rigid branch **(11)** via a second fastening means **(13), characterised in that** the instrument shaft **(4),** which is suitable for transferring an actuation movement and a pivoting movement about a joint axis **(26)** running perpendicularly to the longitudinal axis **(25)** and perpendicularly to the axes of the fastening means **(13, 14, 15, 16),** consists of two branches **(11, 12),** which at the distal end **(5)** have a gripper device **(3)** and at the proximal end **(8)** have an actuation device **(2),** the one branch **(12)** being displaceable relative to the other branch **(11),** and both branches **(11, 12)** having a joint mechanism **(24, 24'),** which is surrounded by a blocking element **(27),** which is formed as a sleeve **(36).**

2. The surgical instrument **(1)** according to claim 1, **characterised in that** the two branches **(11, 12)** of the instrument shaft **(4)** are each formed of a proximal branch part and a distal branch part **(11.1, 11.2, 12.1, 12.2),** the two proximal branch parts **(11.1, 12.1)** being connected to a hand grip element **(9, 10)** and the two distal branch parts **(11.2, 12.2)** being connected to a gripper element **(6, 7).**

3. The surgical instrument **(1)** according to claim 2, **characterised in that** a joint mechanism **(24, 24')** is arranged between each of the branch parts **(11.1, 11.2, 12.1, 12.2)** and is surrounded by a blocking element **(27),** and, once the blocking element **(27)** has been displaced from the blocking position **(28)** into a release position **(29, 29'),** a pivoting movement of the actuation device **(2)** is made possible, in order to pivot the actuation device **(2)** with the gripping device **(3)** lying in one plane out of the common longitudinal axis **(25)** in articulated fashion.

4. The surgical instrument **(1)** according to claim 3, **characterised in that** the joint mechanism **(24, 24')** comprises a joint **(20, 21),** which on the proximal or distal side of the branch part **(11.1, 11.2, 12.1, 12.2)** has a tongue **(30, 30')** and on the opposite distal or proximal side of the branch part **(11.1, 11.2, 12.1, 12.2)** has a corresponding groove **(31, 31'),** which engage in one another and are connected via a fastening means **(26, 26').**

5. The surgical instrument **(1)** according to claim 3, **characterised in that** the blocking element **(27)** forms a sleeve **(36)** which has an opening **(32),** the opening **(32)** in the sleeve **(36)** corresponding with the outer shape **(44)** of the instrument shaft **(4)** and enabling a displacement of the displaceable branch **(12),** in the blocking position **(28)** of the joint **(20, 21),** through the blocking element **(27),** in both directions of the longitudinal axis **(25).**

6. The surgical instrument **(1)** according to claim 5, **characterised in that** the opening **(32)** in the sleeve **(36)** has detent means **(42)** which correspond to detent means **(45)** of the instrument shaft **(4)** in the blocking position **(28)** and release position **(29, 29')** of the blocking element **(27).**

7. The surgical instrument **(1)** according to claim 6, **characterised in that** the detent means **(42)** form protuberances in the sleeve **(36)** and the detent means **(45)** form indentations in the instrument shaft **(4),** or the sleeve **(36)** has indentations and the instrument shaft **(4)** has protuberances.

8. The surgical instrument **(1)** according to claim 5, **characterised in that** the sleeve **(36)** is made of plastics material.

## Revendications

1. Instrument chirurgical **(1)** pour l'occlusion de parties organiques de forme tubulaire du corps, composé d'une tige d'instrument en deux parties **(4),** d'un système de préhension **(3),** d'un système d'actionnement **(2),** le système d'actionnement **(2)** étant composé de deux éléments poignées **(9, 10)** et le premier élément poignée **(9)** ayant une conformation monobloc avec la branche rigide **(11)** et rigide et le second élément poignée **(10)** étant disposé de manière mobile au niveau de la branche rigide **(11)** par l'intermédiaire d'un premier moyen de fixation **(15)** est étant connecté de manière mobile par un second élément de fixation **(16)** à la branche déplaçable **(12)** et le système d'actionnement **(2)** présentant, à proximité des éléments poignées **(9, 10),** un dispositif d'arrêt **(19)** qui contient des cliquets (**17**, **18**) présentant des dents d'enclenchement **(22, 23)** et le système de préhension **(3)** présentant deux éléments de préhension **(6, 7),** un premier élément de préhension **(7)** ayant une conformation monobloc avec la branche déplaçable **(12)** et rigide et le second élément de préhension **(6)** étant disposé de manière mobile par l'intermédiaire d'un premier moyen de fixation **(14)** au niveau de la branche déplaçable **(12)** et étant connecté de manière mobile par un second moyen de fixation **(13)** à la branche rigide **(11), caractérisé en ce que** la tige d'instrument **(4),** qui est apte à transférer un mouvement d'actionnement et un mouvement de pivotement autour d'un axe d'articulation **(26)** s'étendant perpendiculairement à l'axe longitudinal **(25)** et perpendiculairement aux axes des moyens de fixation **(13, 14, 15, 16),** est composé de deux branches **(11, 12)** qui présentent à l'extrémité distale **(5)** un système de préhension **(3)** et à l'extrémité proximale **(8)** un système d'actionnement **(2),** une branche **(12)** étant déplaçable par rapport à l'autre branche **(11)** et les deux branches **(11, 12)** présentant un mécanisme d'articulation **(24, 24')** qui est entouré par un élément de blocage **(27)** qui se présente sous forme d'un manchon **(36).**

2. Instrument chirurgical **(1)** selon la revendication 1, **caractérisé en ce que** les deux branches **(11, 12)** de la tige d'instrument **(4)** sont constituées respectivement d'un élément de branche proximal et distal **(11.1, 11.2, 12.1, 12.2),** les deux éléments de branche proximaux **(11.1, 12.1)** étant connectés à un élément poignée **(9, 10)** et les deux éléments de branches distaux **(11.2, 12.2)** étant connectés à un élément de préhension **(6, 7).**

3. Instrument chirurgical **(1)** selon la revendication 2, **caractérisé en ce que**, entre les éléments de branche **(11.1, 11.2, 12.1, 12.2),** est disposé respectivement un mécanisme d'articulation **(24, 24')** qui est entouré d'un élément de blocage **(27),** sachant que, après le décalage de l'élément de blocage **(27)** de la position de blocage **(28)** vers une position de déblocage **(29, 29'),** un mouvement de pivotement du système d'actionnement **(2)** devient possible pour pivoter de manière articulée le système d'actionnement **(2)** avec le système de préhension se trouvant dans un plan **(3)** depuis l'axe longitudinal commun **(25).**

4. Instrument chirurgical **(1)** selon la revendication 3, **caractérisé en ce que** le mécanisme d'articulation **(24, 24')** comprend une articulation **(20, 21)** qui présente un ressort **(30, 30')** du côté proximal et distal de l'élément de branche **(11.1, 11.2, 12.1, 12.2)** et, du côté distal ou proximal composé de l'élément de branche **(11.1, 11.2, 12.1, 12.2),** une rainure correspondante **(31, 31'),** lesquels ressort et rainure s'engrènent l'un dans l'autre et sont connectés par un moyen de fixation **(26, 26').**

5. Instrument chirurgical **(1)** selon la revendication 3, **caractérisé en ce que** l'élément de blocage **(27)** constitue un manchon **(36)** qui présente une ouverture **(32),** l'ouverture **(32)** du manchon **(36)** qui correspond à la forme extérieure **(44)** de la tige d'instrument **(4)** et qui permet un décalage de la branche déplaçable **(12),** en position de blocage **(28)** de l'articulation **(20, 21),** à travers l'élément de blocage **(27)** dans les deux sens de l'axe longitudinal **(25).**

6. Instrument chirurgical **(1)** selon la revendication 5, **caractérisé en ce que** l'ouverture **(32)** du manchon **(36)** présente des moyens d'enclenchement **(42)** qui correspondent à des moyens d'enclenchement **(45)** de la tige d'instrument **(4)** dans la position de blocage **(28)** et de déblocage (29, 29') de l'élément de blocage **(27).**

7. Instrument chirurgical **(1)** selon la revendication 6, **caractérisé en ce que** les moyens d'enclenchement **(42)** constituent dans le manchon **(36)** des protubérances et que les moyens d'enclenchement **(45)** constituent dans la tige d'instrument **(4)** des creux ou que le manchon **(36)** présente des creux et la tige d'instrument **(4)** des protubérances.

8. Instrument chirurgical **(1)** selon la revendication 5, **caractérisé en ce que** le manchon **(36)** est fabriqué en matière plastique.
